# EUROPÄISCHE PATENTSCHRIFT

(11) **EP 1 140 898 B1**
(45) Veröffentlichungstag und Bekanntmachung des Hinweises auf die Patenterteilung: **28.08.2002**
(21) Anmeldenummer: 98966851.2
(22) Anmeldetag: 22.12.1998
(51) Int. Cl.: C07D 401/04, A61K 31/40, C07D 401/06, C07D 209/14, C07D 401/14, C07D 209/16

(54) **AMID- UND HARNSTOFFDERIVATE ALS 5-HT REUPTAKEINHIBITOREN UND ALS 5-HT1B/1D LIGANDEN**
AMIDE AND UREA DERIVATIVES AS 5-HT REUPTAKE INHIBITORS AND AS 5-HT1B/1D LIGANDS
DERIVES D'AMIDE ET D'UREE S'UTILISANT COMME INHIBITEURS DE REABSORPTION DE 5-HT ET COMME LIGANDS DE 5-HT1B/1D

(43) Veröffentlichungstag der Anmeldung: 10.10.2001
(73) Patentinhaber: MERCK PATENT GmbH, 64293 Darmstadt (DE)
(72) Erfinder: MATZEN, Lisa, D-55131 Mainz (DE); BÖTTCHER, Henning, D-64287 Darmstadt (DE); VAN AMSTERDAM, Christoph, D-64295 Darmstadt (DE); BARTOSZYK, Gerd, D-64331 Weiterstadt (DE); GREINER, Hartmut, D-64331 Weiterstadt (DE); HARTING, Jürgen, D-64287 Darmstadt (DE); RAUTENBERG, Wilfried, D-64354 Reinheim (DE)
(86) Internationale Anmeldenummer: EP9808457
(87) Internationale Veröffentlichungsnummer: WO00037456

(56) Entgegenhaltungen:
- EP-A- 0 548 813
- WO-A-97/14689
- DE-A- 19 615 232

## Beschreibung

Die Erfindung betrifft Amid- und Harnstoff-Derivate der Formel I

R¹-(CH₂)ₙ-(Y)_{q}-(Z)ᵣ-CO-NH-R² I

worin
- R¹: unsubstituiertes oder ein- oder zweifach durch A, AO, OH, Hal, CN, NO₂, NH₂, NHA, NA₂, COA, CONH₂, CONHA, CONA₂, CH₂OH, CH₂OA, CH₂NH₂, CH₂NHA, CH₂NA₂, COOH und/oder COOA substituiertes 3-Indolyl,
- R²: 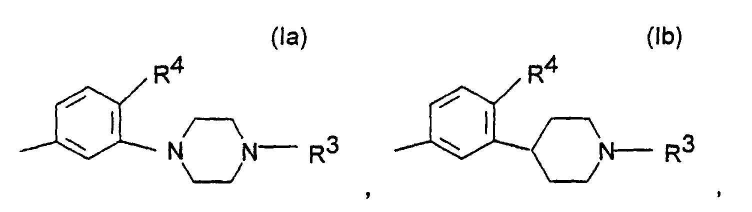 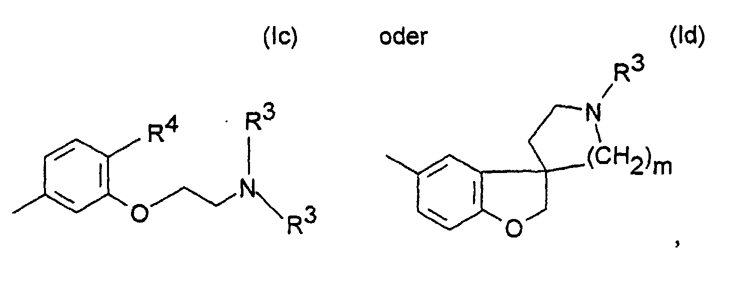
- m: 1 oder 2,
- n: 0, 1, 2, 3 oder 4,
- Y: einen 1,4-Cyclohexylen-, 1,3-Pyrrolidinylen-, 1,4-Piperazinylen- oder 1,4-Piperidinylenring, welcher auch teilweise dehydriert vorliegen kann,
- Z: (CH₂)ₙ oder (CH₂)ₙNH-,
- q: 0 oder 1,
- r: 0 oder 1,
- R³: A,
- R⁴: AO,
- Hal: F, Cl, Br oder 1,
- A: geradkettiges oder verzweigtes Alkyl mit 1-6 C-Atomen,
bedeuten, mit der Maßgabe, dass q und r nicht gleichzeitig 0 sind, sowie deren physiologisch unbedenklichen Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, dass die Verbindungen der Formel I und ihre physiologisch unbedenklichen Säureadditionssalze bei guter Verträglichkeit wertvolle pharmakologische Eigenschaften besitzen, da sie Wirkungen auf das Zentralnervensystem besitzen. Die Verbindungen beeinflussen vor allem die serotoninerge Transmission, indem sie die Wierderaufnahme von Serotonin (5-HT) hemmen und eine starke Affinität zu 5-HT_{1B/1D}-Rezeptoren aufweisen. Durch diese kombinierten Aktivitäten eignen sie sich besonders als Antidepressiva und Anxiolytika.

Die Verbindungen zeigen 5-HT-agonistische und -antagonistische Eigenschaften sowie eine 5-HT Reuptake hemmende Wirkung. Zum in-vitro Nachweis der 5-HT-Wiederaufnahmehemmung wird die synaptosomale Aufnahmehemmung (Wong et al., Neuropsychopharmacology 8 (1993), 23-33) herangezogen. Ex vivo wird diese Eigenschaft im Mäusehimgewebe nach einer Methode von Waldmeier (European J. Pharmacol. 46 (1977), 387-392) untersucht. Die Affinität zu 5-HT_{1B/1D}-Rezeptoren kann beispielsweise nach den von Peroutka et al. (Synapse 3 (1983), 61-66) und Hoyer et al. (European J. Pharmacol. 118 (1985), 1-12) beschriebenen Methoden und die 5-HT_{1B/1D}-antagonistischen Eigenschaften nach einer Methode von Choppin et al. (British Journal of Pharmacology, 114 (1995), 309-314) bestimmt werden.

Ähnliche Verbindungen, die ebenfalls 5-HT1B/D- antagonistische Wirkungen zeigen, sind beispielweise beschrieben in den Patentanmeldungen WO 97/14689 oder WO 97/41802.

Die Verbindungen der Formel I eignen sich daher sowohl in der Veterinärals auch in der Humanmedizin zur Behandlung von Funktionsstörungen des Zentralnervensystems sowie von Entzündungen. Sie können zur Prophylaxe und zur Bekämpfung der Folgen cerebraler Infarktgeschehen (apoplexia cerebri) wie Schlaganfall und cerebraler Ischämien sowie zur Behandlung extrapyramidal-motorischer Nebenwirkungen von Neuroleptika sowie des Morbus Parkinson, zur akuten und symptomatischen Therapie der Alzheimer Krankheit sowie zur Behandlung der amyotrophen Lateralsklerose verwendet werden. Ebenso eignen sie sich als Therapeutika zur Behandlung von Hirn- und Rückenmarkstraumata. Insbesondere sind sie jedoch geeignet als Arzneimittelwirkstoffe für Anxiolytika, Antidepressiva, Antipsychotika, Neuroleptika, Antihypertonika und/oder zur positiven Beeinflussung von Zwangsverhalten (obsessive-compulsive disorder, OCD), Angstzuständen, Panikattacken, Psychosen, Anorexie, wahnhaften Zwangsvorstellungen, Agoraphobie, Migräne, der Alzheimer Krankheit, Schlafstörungen, tardiver Dyskinesien, Lemstörungen, altersabhängiger Erinnerungsstörungen, Essstörungen wie Bulimie, Drogenmissbrauch und/oder Sexualfunktionsstörungen.
Desweiteren sind sie geeignet zur Behandlung von endokrinen Erkrankungen wie Hyperprolactinaemie, ferner bei Vasospasmen, Hypertension und gastrointestinalen Erkrankungen.
Ferner können sie als Zwischenprodukte zur Herstellung weiterer Arzneimittelwirkstoffe eingesetzt werden.

Gegenstand der Erfindung sind Amid- und Harnstoffderivate der Formel I sowie ihre physiologisch unbedenklichen Säureadditionssalze.

Gegenstand der Erfindung sind insbesondere Verbindungen der Formel I, ausgewählt aus der Gruppe
a) N-[2-(5-Fluor-3-indolyl)-ethyl]-N'-[4-methoxy-3-(4-methyl-1-piperazinyl)-phenyl]-harnstoff;
b) 4-(5-Cyan-3-indolyl)-N-[4-methoxy-3-(4-methyl-piperazin-1-yl)-phenyl]-piperidin-1-carboxamid;
c) 4-(6-Fluor-3-indolyl)-N-[4-methoxy-3-(4-methyl-piperazin-1-yl)-phenyl]-piperidin-1-carboxamid;
d) 3-{1-[4-Methoxy-3-(4-methyl-1-piperazinyl)-phenylaminocarbonyl]-4-piperidyl}-indol-5-carboxamid;
e) 4-(5-Fluor-3-indoiyl)-N-[4-methoxy-3-(4-methyl-piperazin-1-yl)-phenyl]-piperidin-1-carboxamid;
f) 4-[2-(3-lndolyl)-ethyl]-N-[4-methoxy-3-(4-methyl-piperazin-1-yl)-phenyl]-piperidin-1-carboxamid;
g) 4-(3-Indolyl)-N-[4-methoxy-3-(4-methyl-piperazin-1-yl)-phenyl]-piperidin-1-carboxamid;
h) 4-(4-Fluor-3-indolyl)-N-[4-methoxy-3-(4-methyl-piperazin-1-yl)-phenyl]-piperidin-1-carboxamid;
i) 4-(7-Methoxy-indol-3-yl)-piperidin-1-carbonsäue[4-methoxy-3-(4-methylpiperazin-1-yl)-phenyl]-amid;
j) 4-[4-(5-Fluor-3-indolyl)-butyl]-piperazin-1-carbonsäure[4-methoxy-3-(4-methyl-piperazin-1-yl)-phenyl]-amid;
k) 4-(5-Cyan-indol-3-yl)-3,6-dihydro-2H-pyridin1-carbonsäure[4-methoxy-3-(4-methyl-piperazin-1-yl)-phenyl]-amid;
l) 3-(5-Fluor-indol-3-yl)-pyrrolidin1-carbonsäure[4-methoxy-3-(4-methylpiperazin-1-yl)-phenyl]-amid;
m) 4-(5-Fluor-indol-3-yl)-cyclohexancarbonsäure[4-methoxy-3-(4-methylpiperazin-1-yl)-phenyl]-amid;
n) N-[2-(5-Hydroxy-3-indolyl)ethyl]-N'-[4-methoxy-3-(4-methyl-1-piperazinyl)-phenyl]-harnstoff;
o) N-{2-[4-(6-Fluor-3-indolyl)-piperidin-1-yl]ethyl}-N'-[4-methoxy-3-(4-methyl-1-piperazinyl)-phenyl]-harnstoff;
p) N-[4-(5-Cyan-3-indolyl)-butyl]-N'-[4-methoxy-3-(4-methyl-1-piperazinyl)-phenyl]-hamstoff;
q) 4-[4-(5-Cyan-3-indolyl)-butyl]-piperazin-1-carbonsäure[4-methoxy-3-(4-methyl-1-piperazinyl)-phenyl]-amid;
r) 4-(5-Fluor-indol-3-yl)-piperidin-1-carbonsäure-(2,3-dihydro-1'methylspiro(benzofuran)-3,4-piperidin)-amid;
s) N-{2-[4-(6-Fluor-3-indolyl)-piperidin-1-yl]ethyl}-N'-[2,3-dihydro-1-methylspiro(benzofuran)-3,4-piperidin]-harnstoff;
sowie deren physiologisch unbedenklichen Salze.

Gegenstand der Erfindung sind dementsprechend die Verbindungen der Formel I sowie ein Verfahren zur Herstellung von Verbindungen der Formel I gemäß Anspruch 1.

Das Verfahren zur Herstellung ist dadurch gekennzeichnet, dass man
a) eine Verbindung der Formel II

   II H₂N-R²

   worin R² die in Anspruch 1 angegebene Bedeutung hat,
   mit einer Verbindung der Formel III

   III R¹-(CH₂)ₙ-(Y)_{q}-(Z)ᵣ-CO-L

   worin L Cl, Br, I, OH oder eine andere reaktionsfähig funktionell abgewandelte OH-Gruppe oder leicht nucleophil substituierbare Abgangsgruppe bedeutet und
   R¹, n, Y, q, Z und r die in Anspruch 1 angegebenen Bedeutungen haben,
   umsetzt,
   oder
b) die Aminkomponente der Formel II

   II H₂N-R²

   mit der Komponente der Formel IV

   IV R¹-(CH₂)ₙ-(Y)_{q}-(Z)ᵣ-H

   worin R¹,R², n, Y, q, Z und r die angegebenen Bedeutungen haben, unter Zusatz von Kupplungsreagenzien wie N,N'-Carbonyldiimidazol, Diphosgen, Triphosgen oder auch Chlorameisensäureestem
   umsetzt,
   und/oder
c) dass man gegebenenfalls einen der Reste R¹, R³ und/oder R⁴ in einen anderen Rest R¹, R³ und/oder R⁴ umwandelt, indem man z.B. eine OA-Gruppe unter Bildung einer OH-Gruppe spaltet und/oder eine CN-, COOH- oder COOA-Gruppe derivatisiert und/oder dass man z.B. ein primäres oder sekundäres N-Atom alkyliert und/oder dass man eine erhaltene Base oder Säure der Formel I durch Behandeln mit einer Säure oder Base in eines ihrer Salze umwandelt.

Gegenstand der Erfindung sind ebenfalls Arzneimittel enthaltend Verbindungen der Formel I und deren physiologisch unbedenklichen Salze mit 5-HT1B/D-antagonistischer und 5-HT-reuptake-hemmender Wirkung.

Gegenstand der Erfindung sind die Verbindungen der Formel I sowie deren Enantiomere sowie Diastereomere und deren Salze.

Für alle Reste, die mehrfach auftreten, wie z.B. A oder R³, gilt, dass deren Bedeutungen unabhängig voneinander sind.

Der Rest A bedeutet Alkyl und hat 1 bis 6, vorzugsweise 1, 2, 3 oder 4, insbesondere 1 oder 2 C-Atome. Alkyl bedeutet daher insbesondere z.B. Methyl, weiterhin Ethyl, n-Propyl, Isopropyl, n-Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl.

OA ist vorzugsweise Methoxy, ferner auch Ethoxy, n-Propoxy, Isopropoxy, n-Butoxy, Isobutoxy, sek.-Butoxy oder tert.-Butoxy.

NHA ist vorzugsweise Methylamino, ferner Ethylamino, Isopropylamino, n-Butylamino, Isobutylamino, sek.-Butylamino oder tert.-Butylamino. NA₂ bedeutet vorzugsweise Dimethylamino, ferner N-Ethyl-N-methylamino, Diethylamino, Di-n-propylamino, Diisopropylamino oder Di-n-butylamino. Daraus resultierend bedeutet CO-NHA vorzugsweise N-Methylcarbamoyl oder N-Ethyl-carbamoyl; CO-NA₂ vorzugsweise N,N-Dimethylcarbamoyl oder N,N-Diethylcarbamoyl.

Hal bedeutet Fluor, Chlor, Brom oder lod, insbesondere Fluor oder Chlor.

n bedeutet 0, 1, 2, 3 oder 4, insbesondere 0, 2 oder 4.

Der Rest R³ bedeutet jeweils unabhängig voneinander A, wobei die beiden Reste R³ in Formel (lc) gleich oder verschieden sein können. Vorzugsweise sind sie beide gleich und bedeuten insbesondere bevorzugt Methyl.

Y bedeutet vorzugsweise einen 1,4-Piperazinylen- oder 1,4-Piperidinylenring, welcher auch teilweise dehydriert vorliegen kann und vorzugsweise dann einen 1,4-substituierten 3,6-Dihydro-2H-pyridinring darstellt, ferner auch einen 1,3-Pyrrolidinylen- oder 1,4-Cyclohexylenring.

R¹ bedeutet vorzugsweise unsubsitituiertes oder ein- oder zweifach, insbesondere jedoch einfach, durch Hal, CN, A, AO, CONH₂, CONHA, CONA₂, COOH, COOA, CH₂Ar, CH₂OAr und/oder CH₂NA₂ substituiertes 2- oder 3-Indolyl, wobei das 3-Indolyl besonders bevorzugt ist. Vorzugsweise ist der Indolrest in 5-Stellung, ferner auch in der 4-, 6- oder 7-Stellung substituiert.

R¹ bedeutet daher besonders bevorzugt 3-Indolyl, 5- oder 6-Methyl-indol-3-yl, 5- oder 6-Methoxy-indol-3-yl, 5- oder 6-Hydroxy-indol-3-yl, 4-, 5- oder 6-Fluor-indol-2-yl, 4-, 5- oder 6-Fluor-indol-3-yl, 5- oder 6-Cyan-indol-3-yl, 5- oder 6-Chlor-indol-3-yl, 5- oder 6-Carboxy-indol-3-yl, 5- oder 6-Methoxycarbonyl-indol-3-yl, 5- oder 6-Hydroxy-indoly-3-yl, 5- oder 6-Hydroxymethyl-indol-3-yl, 5- oder 6-Aminomethyl-indol-2-yl, 5- oder 6-Aminomethyl-indol-3-yl, ferner 5- oder 6-Brom-indol-3-yl, 5- oder 6-Ethyl-indol-3-yl, 5- oder 6-Isopropyl-indol-3-yl, 5- oder 6-Dimethylamino-indol-3-yl oder 5- oder 6-Ethoxy-indol-3-yl.

R² bedeutet (la), (lb), (lc) oder (ld). Ganz besonders bevorzugt sind dabei Verbindungen, die als R² die Gruppen (la), (lc) oder (ld) enthalten.

m bedeutet 1 oder 2, vorzugsweise 2.

Z bedeutet (CH₂)ₙ oder (CH₂)ₙNH-, wobei n vorzugsweise 2 oder 4 ist.

q und r bedeuten jeweils 0 oder 1, wobei die Maßgabe gilt, dass q und r nicht beide gleichzeitig 0 sein können.

Für die gesamte Erfindung gilt, dass sämtliche Reste, die in einem Molekül mehrfach auftreten können, gleich oder verschieden sein können, d.h. unabhängig voneinander sind.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nachstehenden Formeln I1 bis I12 ausgedrückt werden, die der Formel I entsprechen und worin die nicht näher bezeichneten Reste die bei der Formel I angegebene Bedeutung haben, worin jedoch

| | |
|---|---|
| in l1 | R² die Gruppe (la) ist; |
| | |
| in l2 | R² eine in I1 angegebene Bedeutung hat und R⁴ ein Methoxy- und R³ ein Methyl-Rest ist; |
| | |
| in l3 | R² die Gruppe (ld) ist; |
| | |
| in l4 | R² die in I3 angegebene Bedeutung hat und der Rest R³ eine Methylgruppe darstellt; |
| | |
| in l5 | R² die Gruppe (lb) oder (lc) ist; |
| | |
| in l6 | q = 1 und r = 0 ist; |
| | |
| in l7 | R¹ ein einfach durch CN, F, OA, CONH₂ oder OH substituiertes indol-3-yl ist; |
| | |
| in l8 | q = 0 und r = 1 ist; |
| | |
| in l9 | q und r die in I6 angegebene Bedeutung haben und Y einen 1,4-Piperidinylenring bedeutet; |
| | |
| in l10 | q und r die in I6 angegebene Bedeutun haben und Y einen 1,4-Cyclohexylen- oder 1,4-Piperazinylenring bedeutet; |
| | |
| in l11 | q und r die in I8 angegebene Bedeutung haben und n 2 oder 4 ist; |
| | |
| in l12 | q = r = 1 ist. |

Die Verbindungen der Formel I und auch die Ausgangsstoffe zu ihrer Herstellung werden im übrigen nach an sich bekannten Methoden hergestellt, wie sie in der Literatur (z.B. in Standardwerken wie Houben-Weyl, Methoden der Organischen Chemie, Georg Thieme Verlag, Stuttgart; Organic Reactions, John Wiley & Sons, Inc., New York;) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, dass man sie aus dem Reaktionsgemisch nicht isoliert, sondern sofort weiter zu den Verbindungen der Formel I umsetzt. Andererseits ist es möglich, die Reaktion stufenweise durchzuführen.

In den Verbindungen der Formel III ist der Rest L vorzugsweise Cl oder Br; er kann jedoch auch I, OH oder auch bevorzugt eine reaktionsfähig funktionell abgewandelte OH-Gruppe bedeuten, insbesondere Alkylsulfonyloxy mit 1-6 (z.B. Methansulfonyloxy) oder Arylsulfonyloxy mit 6-10 C-Atomen (z.B. Benzolsulfonyloxy, p-Toluolsulfonyloxy, 1- oder 2-Naphthalinsulfonyloxy) oder auch Trichlomethoxy, Alkoxy, wie z.B. Methoxy, Ethoxy, Propoxy oder Butoxy, femer auch Phenoxy.

Die Verbindungen der Formel I können vorzugsweise erhalten werden, indem man Verbindungen der Formel II mit Verbindungen der Formel III umsetzt.

Die Ausgangsstoffe der Formeln II und III sind in der Regel bekannt; die nicht bekannten Verbindungen der Formeln II und III können leicht analog zu den bekannten Verbindungen hergestellt werden.

Die Verbindungen der Formel III können beispielweise auch durch elektrophile oder in bestimmten Fällen auch nukleophile aromatische Substitutionen aus bekannten Verbindungen hergestellt werden.
Als Ausgangssubstanz dient dabei oftmals die entsprechende Indol-3-alkansäure (herstellbar analog einer Japp-Klingemann-Typ Fischer Indolsynthese, vgl. dazu Böttcher et al., J. Med. Chem. 1992, 35, 4020-4026 oder lyer et al., J. Chem. Soc. Perkin Trans. II 1973, 872-878), welche je nach Bedarf weiter reduziert und substituiert werden kann.

Die Umsetzung der Verbindungen II und III verläuft nach Methoden, wie sie für die Alkylierung bzw. Acylierung von Aminen aus der Literatur bekannt sind. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, N-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetallhydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses Piperazin-Derivates der Formel II kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Gegebenenfalls ist es notwendig, vor der Durchführung dieser Reaktion weitere enthaltene Aminogruppen durch Einführung von geeigneten Schutzgruppen vor einer Alkylierung oder Acylierung zu schützen. Der Ausdruck Aminoschutzgruppe ist allgemein bekannt und bezieht sich auf Gruppen, die geeignet sind, eine Aminogruppe vor chemischen Umsetzungen zu schützen, die aber leicht entfernbar sind, nachdem die gewünschte chemische Reaktion an einer anderen Stelle des Moleküls durchgeführt worden ist. Da dem Fachmann solche Schutzgruppen sowie die Einführung und Abspaltung dieser aus zahlreichen Literaturstellen und Lehrbüchern wohl bekannt sind, muss an dieser Stelle darauf nicht näher eingegangen werden.

Desweiteren kann man Verbindungen der Formel I herstellen, indem man Amine der Formel II mit einer Komponte der Formel IV enthaltend den Rest R¹ umsetzt.

Die jeweiligen Komponenten sind in der Regel bekannt oder können wie schon beschrieben nach bekannten Verfahren hergestellt werden.

Die Herstellung von Verbindungen der Formel II, in welchen R² den Rest la (Piperazinderivate) bedeutet, ist beispielsweise auch beschrieben von Clitherow,J.W. et al., im J. Med.Chem 1994, 37 (15), 2253-2257. Die Verbindungen der Formel II, in welchen R² lb (Piperidinderivate) bedeutet, sind ebenfalls bekannt, beispielweise aus der WO 96/31508.
Die Spiroverbindungen der Formel II mit R² gleich Id sind aus der WO 96/19477 und die Verbindungen der Formel II, worin R² den Rest lc bedeutet, aus der WO 95/26328 bekannt.

Die Verbindungen der Formel II und IV werden nun durch Zuhilfenahme von Kupplungsreagenzien, wie z.B. N,N'-Carbonyldiimidazol, Diphosgen oder Triphosgen oder auch Chlorameisensäureester, durchgeführt. Diese Synthese wird nach den üblichen Bedingungen einer Acylierung, wie schon beschrieben, durchgeführt. Vorzugsweise wird hier diese Verknüpfung mit N,N-Carbonyldiimidazol, Triethylamin und Acetonitril als indifferentes Lösungsmittel bei Raumtemperatur durchgeführt.

Darüber hinaus ist es möglich, bestimmte Reduktionen durch Verwendung von H₂-Gas unter katalytischer Wirkung von Übergangsmetallen, wie z.B. Raney-Ni oder Pd durchzuführen. Man kann auf diese Weise z.B. Cl, Br, I, SH oder in bestimmten Fällen auch OH-Gruppen durch Wasserstoff ersetzen. Ebenso können Nitrogruppen durch katalytische Hydrierung mit Pd/H₂ in Methanol in NH₂-Gruppen umgewandelt werden.

Verbindungen, die sonst der Formel I entsprechen, aber anstelle eines oder mehrerer H-Atome eine oder mehrere solvolysierbare Gruppe(n) enthalten, können zu den Verbindungen der Formel I solvolysiert, insbesondere hydrolysiert werden.

Weiterhin kann man eine Verbindung der Formel I nach an sich bekannten Methoden in eine andere Verbindung der Formel I umwandeln.

Verbindungen der Formel I, worin R¹ einen durch CONH₂, CONHA oder CONA₂ substituierten Indol-Rest bedeutet, können durch Derivatisierung entsprechender substituierter Verbindungen der Formel durch partielle Hydrolyse erhalten werden. Ferner ist es möglich, Cyan-substituierte Verbindungen der Formel I zunächst zu Säuren zu hydrolysieren und die Säuren mit primären oder sekundären Aminen zu amidieren. Bevorzugt ist die Umsetzung der freien Carbonsäure mit dem Amin unter den Bedingungen einer Peptidsynthese. Diese Reaktion gelingt vorzugsweise in Gegenwart eines Dehydratisierungsmittels, z.B. eines Carbodiimids wie Dicyclohexylcarbodiimid oder N-(3-Dimethylaminopropyl)-N-ethyl-carbodiimid, ferner Propanphosphonsäureanhydrid (vgl. Angew. Chem. 92, 129 (1980)), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40°, vorzugsweise zwischen 0 und 30°. Anstelle der Säure bzw. des Amids können auch reaktionsfähige Derivate dieser Stoffe in die Reaktion eingesetzt werden, z.B. solche, in denen reaktive Gruppen intermediär durch Schutzgruppen blockiert sind. Die Säuren können auch in Form ihrer aktivierten Ester verwendet werden, die zweckmäßig in situ gebildet werden, z.B. durch Zusatz von 1-Hydroxybenztriazol oder N-Hydroxysuccinimid.

Besonders günstig ist es aber auch in umgekehrter Weise, durch Wasserabspaltung, ausgehend von den Amiden, z.B. mittels Trichloracetylchlorid/Et₃N [Synthesis (2), 184 (1985)] oder mit POCl₃ (J. Org. Chem. 26, 1003 (1961)), die Nitrile herzustellen.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure; Benzolsulfonsäure, p-Toluolsulfonsäure, Naphthalin-monound -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden, sofern keine weiteren aciden Gruppen im Molekül vorliegen. In jenen Fällen, wo die Verbindungen der Formel I über freie Säuregruppen verfügen, kann durch Behandlung mit Basen ebenfalls eine Salzbildung erreicht werden. Als Basen eignen sich Alkalimetallhydroxide, Erdalkalimetallhydroxide oder organische Basen in Form von primären, sekundären oder tertiären Aminen.

Gegenstand der Erfindung ist ferner die Verwendung der Verbindung der Formel I und/oder ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind femer Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- und Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositoren, zur parenteralen Applikation Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenden Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der therapeutischen Behandlung des menschlichen oder tierischen Körpers und bei der Bekämpfung von Krankheiten verwendet werden. Sie eignen sich zur Behandlung von Erkrankungen des Zentralnervensystems wie Spannungszuständen, Depressionen, Angstzuständen, Schizophrenie, Magen-Darm-Trakt-Störungen, Übelkeit, tardiven Dyskinesien, Parkinsonismus und/oder Psychosen und von Nebenwirkungen bei der Behandlung der Hypertonie (z.B. mit α-Methytdopa). Ferner können die Verbindungen in der Endokrinologie und Gynäkologie Verwendung finden, z.B. zur Therapie von Akromegalie, Hypogonadismus, sekundärer Amenorrhoe, prämenstruellem Syndrom, unerwünschter puerperaler Laktation, weiterhin zur Prophylaxe und Therapie cerebraler Störungen (z.B. Migräne), insbesondere in der Geriatrie ähnlich wie gewisse Ergot-Alkaloide.
Insbesondere bevorzugt können sie auch als Therapeutika zur Bekämpfung der Folgen cerebraler Infarktgeschehen (apoplexia cerebri), wie Schlaganfall und cerebraler Ischämien und zur Behandlung von Hirnund Rückenmarkstraumata eingesetzt werden.

Insbesondere sind sie jedoch geeignet als Arnzeimittelwirkstoffe für Anxiolytika, Antidepressiva, Antipsychotika und/oder zur positiven Beeinflussung von Zwangsverhalten (OCD), Schlafstörungen, tardiver Dyskinesien, Lernstörungen, altersabhängiger Erinnerungsstörungen, Essstörungen wie Bulimie und/oder Sexualfunktionsstörungen.

Dabei werden die erfindungsgemäßen Substanzen in der Regel in Analogie zu bekannten Präparaten verabreicht, vorzugsweise in Dosierungen zwischen etwa 0,1 und 500 mg, insbesondere zwischen 5 und 300 mg pro Dosierungseinheit. Die tägliche Dosierung liegt vorzugsweise zwischen etwa 0,01 und 250 mg/kg, insbesondere zwischen 0,02 und 100 mg/kg Körpergewicht

Dabei werden die erfindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachstehenden Beispielen bedeutet "übliche Aufarbeitung": Man entfernt, falls erforderlich, das Lösungsmittel, gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, filtriert, engt ein und reinigt durch Chromatographie an Kieselgel und/oder durch Kristallisation.

### Beispiel 1

Eine Lösung von 4-Methoxy-3-(4-methyl-piperazin-1-yl)-anilin-dihydrochlorid (2.65 g; 9 mmol), Triethylamin (4.5 ml; 31.5 mmol) und N,N'-Carbonyldiimidazol (1.6 g; 10 mmol) in 125 ml Acetonitril wird bei Raumtemperatur 3 Stunden lang gerührt. Dazu gibt man eine Suspension von 2.0 g (9 mmol) 5-Fluor-3-piperidin-4-yl-1H-indol und 1.3 ml (9 mmol) Triethylamin in 125 ml Acetonitril und rührt noch weitere 12 Stunden bei Raumtemperatur. Nach der üblichen Aufarbeitung löst man den Rückstand in Aceton und fällt mit 1 N HCI das Hydrochlorid. Durch Umkristallisation aus Ethanol/Diethylether erhält man 4-(5-Fluor-1H-indol-3-yl)-piperidin-1-carbonsäure[4-methoxy-3-(4-methyl-piperazin-1-yl)-phenyl]-amidhydrochlorid

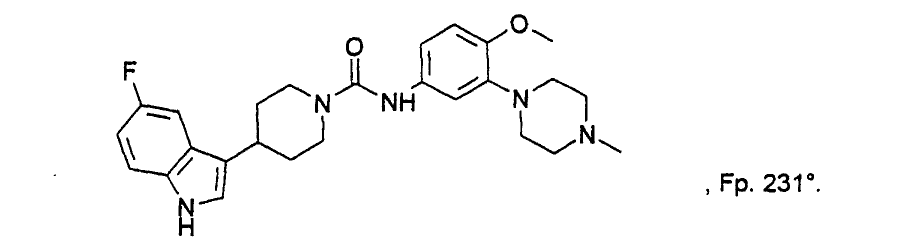

Analog werden hergestellt:
4-(3-Indolyl)-N-[4-methoxy-3-(4-methyl-1-piperazinyl)-phenyl]-piperidin1-carboxamid-dihydrochlorid, Fp. 204°;
4-(5-Fluor-1H-indol-3-yl)-piperidin-1-carbonsäure[4-methoxy-3-(N,N'dimethylaminoethoxy)-phenyl]-amid;
4-(5-Fluor-1H-indol-3-yl)-piperidin-1-carbonsäure[4-methoxy-3-(4-methylpiperidin-1-yl)-phenyl]-amid-hydrochlorid;
4-(5-Cyan-3-indolyl)-N-[4-methoxy-3-(4-methyl-1-piperazinyl)-phenyl]-piperidin1-carboxamid, Fp. 194-195°;
4-(6-Fluor-3-indolyl)-N-[4-methoxy-3-(4-methyl-1-piperazinyl)-phenyl]-piperidin1-carboxamid, Fp. 135-137°;
3-{1-[4-Methoxy-3-(4-methyl-1-piperazinyl)-phenylaminocarbonyl]-4-piperidyl}-indol-5-carboxamid, Fp. 176-178°;
4-(4-Fluor-3-indolyl)-N-[4-methoxy-3-(4-methyl-1-piperazinyl)-phenyl]-piperidin1-carboxamid-dihydrochlorid, Fp. >205° (Zers.);
4-(4-Fluor-3-indolyl)-N-[4-methoxy-3-(4-methyl-1-piperidinyl)-phenyl]-piperidin1-carboxamid-dihydrochlorid;
4-(7-Methoxy-1H-indol-3-yl)-piperidin-1-carbonsäure[4-methoxy-3-(4-methyl-piperazin-1-yl)-phenyl]-amid, Fp. 219-222°;
4-[2-(3-Indolyl)-ethyl]-N-[4-methoxy-3-(4-methyl-1-piperazinyl)-phenyl]-piperidinl-carboxamid, Fp. 200-202°;
4-[2-(3-Indolyl)-ethyl]-N-[4-methoxy-3-(N,N'-dimethylaminoethoxy)-phenyl]-piperidin-1-carboxamid;
4-[2-(3-lndolyl)-ethyl]-N-[4-methoxy-3-(4-methyl-1-piperidinyl)-phenyl]-piperidin1-carboxamid;
4-(5-Cyan-3-indolyl)-3,6-dihydro-2H-pyridin-1-carbonsäure[4-methoxy-3-(4-methyl-1-piperazinyl)-phenyl]-amid, Fp. 223-225°;
4-[4-(5-Fluor-indol-3-yl)butyl]-piperazin-1-carbonsäure[4-methoxy-3-(4-methyl-1-piperazinyl)-phenyl]-amid-dihydrochlorid, Fp. >220° (Zers.);
3-(5-Fluor-indol-3-yl)-pyrrolidin-1-carbonsäure[4-methoxy-3-(4-methyl-1-piperidinyl)-phenyl]-amid, Fp. 210-213°;
4-(5-Fluor-indol-3-yl)-piperidin-1-carbonsäure-(2,3-dihydro-1'methylspiro(benzofuran)-3,4-piperidin)-amid;
4-[4-(5-Cyan-3-indolyl)-butyl]-piperazin-1-carbonsäure[4-methoxy-3-(4-methyl-1-piperazinyl)-phenyl]-amid;
4-[4-(5-Cyan-3-indolyl)-butyl]-piperazin-1-carbonsäure[4-methoxy-3-(4-methyl-1-piperidinyl)-phenyl]-amid;
4-(5-Cyan-1H-indol-3-yl)-piperidin-1-carbonsäure-[4-methoxy-3-(N,N'dimethylaminoethoxy)-phenyl]-amid;
4-(3-Indolyl)-piperidin-1-carbonsäure-[4-methoxy-3-(N,N'dimethylaminoethoxy)-phenyl]-amid;
4-[4-(5-Fluor-1H-indol-3-yl)butyl]-piperazin-1-carbonsäure-[4-methoxy-3-(N,N'-dimethylaminoethoxy)-phenyl]-amid;
4-[4-(5-Cyan-1H-indol-3-yl)butyl]-piperazin-1-carbonsäure-[4-methoxy-3-(N,N'-dimethylaminoethoxy)-phenyl]-amid;
4-(5-Cyan-1H-indol-3-yl)-piperidin-1-carbonsäure-(2,3-dihydro-1'methylspiro(benzofuran)-3,4-piperidin)-amid 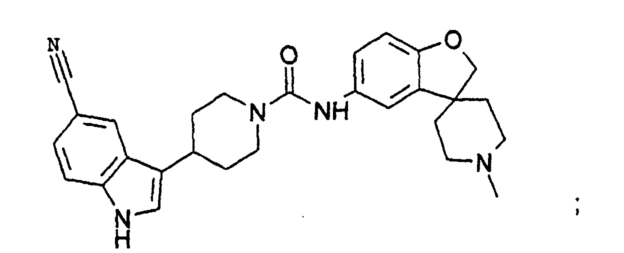
4-(3-Indolyl)-piperidin-1-carbonsäure-(2,3-dihydro-1'-methylspiro(benzofuran)-3,4-piperidin)-amid;
4-[4-(5-Fluor-1H-indol-3-yl)butyl]-piperidin-1-carbonsäure-(2,3-dihydro-1'methylspiro(benzofuran)-3,4-piperidin)-amid;
4-[4-(5-Cyan-1H-indol-3-yl)butyl]-piperidin-1-carbonsäure-(2,3-dihydro-1'methylspiro(benzofuran)-3,4-piperidin)-amid;
4-[3-(1H-Indol-3-yl)propyl]-piperidin-1-carbonsäure-(2,3-dihydro-1'methylspiro(benzofuran)-3,4-piperidin)-amid.

### Beispiel 2

Analog Beispiel 1 erhält man durch Umsetzung von 1.17 g (4 mmol) 4-Methoxy-3-(4-methyl-piperazin-1-yl)-anilin-dihydrochlorid, 2.0 ml (14 mmol) Triethylamin und 714 mg (4.4 mmol) N,N'-Carbonyldiimidazol in 35 ml Acetonitril mit 859 mg (4 mmol) 5-Fluortryptamin-hydrochlorid und 1.1 ml (8 mmol) Triethylamin in 35 ml Acetonitril und anschließender Umkristallisation aus Ethylacetat/ Petrolether den N-[2-(5-Fluor-3-indolyl)ethyl]-N'-[4-methoxy-3-(4-methyl-1-piperazinyl)-phenyl]-hamstoff

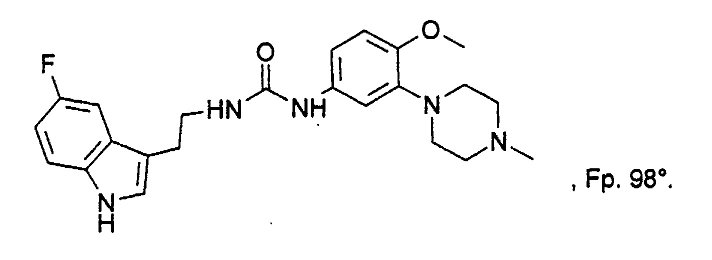

Analog werden hergestellt:
N-[2-(5-Hydroxy-3-indolyl)ethyl]-N'-[4-methoxy-3-(4-methyl-1-piperazinyl)-phenyl]-harnstoff;
N-[4-(5-Cyan-3-indolyl)butyl]-N'-[4-methoxy-3-(4-methyl-1-piperazinyl)-phenyl]-harnstoff;
N-[2-(5-Fluor-3-indolyl)ethyl]-N'-[4-methoxy-3-(4-methyl-1-piperidinyl)-phenyl]-harnstoff;
N-[2-(5-Fluor-3-indolyl)ethyl]-N'-[4-methoxy-3-(N,N'-4-dimethylaminoethoxy)-phenyl]-hamstoff;
N-{2-[4-(6-Fluor-3-indolyl)-piperidin-1-yl]ethyl}-N'-[2,3-dihydro-1'methylspiro(benzofuran)-3,4-piperidin]-harnstoff;
N-{2-[4-(6-Fluor-3-indolyl)-piperidin-1-yl]ethyl}-N'-[4-methoxy-3-(4-methyl-1-piperazinyl)-phenyl]-harnstoff, Fp. 189-192;
N-{2-[4-(6-Fluor-3-indolyl)-piperidin-1-yl]ethyl}-N'-[4-methoxy-3-(4-methyl-1-piperidinyl)-phenyl]-hamstoff;
N-{2-[4-(6-Fluor-3-indolyl)-piperidin-1-yl]ethyl}-N'-[4-methoxy-3-(N,N'-4-dimethylaminoethoxy)-phenyl]-hamstoff.

### Beispiel 3

Zu einer Mischung von 314 mg (1.2 mmol) 4-(5-Fluor-1H-indol-3-yl)-cyclohexancarbonsäure und 265 mg (1.2 mmol) 4-Methoxy-3-(4-methylpiperazin-1-yl)-anilin in 40 ml Dichlormethan gibt man 173 µl (1.2 mmol) Triethylamin und 230 mg (1.2 mmol) 1-Ethyl-3-(3-dimethylaminopropyl)-carbodiimid-hydrochlorid. Man lässt zunächst eine Stunde lang bei 0° rühren, anschließend bei 12 h bei Raumtemperatur. Das Reaktionsgemisch wird eingeengt, der Rückstand wird in Ethylacetat aufgenommen, mit Wasser, 5% Citronensäure und ges. Natriumbicarbonatlösung gewaschen, getrocknet und eingedampft. Nach einer säulenchromatographischen Trennung erhält man das 4-(5-Fluor-1H-indol-3-yl)-cyclohexansäure[4-methoxy-3-(4-methyl-piperazin-1-yl)-phenyl]-amid.

### Analog werden hergestellt:

4-(5-Cyan-1H-indol-3-yl)-cyclohexansäure[4-methoxy-3-(4-methylpiperazin-1-yl)-phenyl]-amid,
4-(6-Fluor-1H-indol-3-yl)-cyclohexansäure[4-methoxy-3-(4-methylpiperazin-1-yl)-phenyl]-amid,
4-(5-Fluor-1H-indol-3-yl)-cyclohexansäure[4-methoxy-3-(4-methylpiperidin-1-yl)-phenyl]-amid,
4-(5-Cyan-1H-indol-3-yl)-cyclohexansäure[4-methoxy-3-(4-methylpiperidin-1-yl)-phenyl]-amid,
4-(6-Fluor-1H-indol-3-yl)-cyclohexansäure[4-methoxy-3-(4-methylpiperidin-1-yl)-phenyl]-amid,
4-(5-Fluor-1H-indol-3-yl)-cyclohexansäure[4-methoxy-3-(N,N'dimethylaminoethoxy)-phenyl]-amid,
2-[4-(5-Fluor-1H-indol-3-yl)-piperidin-1-yl]-N-[4-methoxy-3-(4-methylpiperazin-1-yl)-phenyl]-acetamid,
2-[4-(5-Fluor-1H-indol-3-yl)-piperidin-1-yl]-N-[3-(2-dimethylamino-ethoxy)-4-methoxy-3-phenyl]-acetamid,
2-[4-(1H-indol-3-yl)-piperidin-1-yl]-N-[4-methoxy-3-(4-methyl-piperazin-1-yl)-phenyl]-acetamid,
2-[4-(1H-Indol-3-yl)-piperidin-1-yl]-N-[3-(2-dimethylamino-ethoxy)-4-methoxy-3-phenyl]-acetamid.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat in 3 I zweifach destilliertem Wasser wird mit 2 n Salzsäure auf pH 6,5 eingestellt, steril filtriert, in Injektionsgläser abgefüllt, lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9.38 g NaH₂PO₄ x 2 H₂O, 28.48 g NaH₂PO₄ x 12 H₂O und 0.1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1.2 kg Kartoffelstärke, 0.2 kg Talk und 0.1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird in Ampullen abgefüllt, unter aseptischen Bedingungen lyophilisiert und steril verschlossen. Jede Ampulle enthält 10 mg Wirkstoff.

## Patentansprüche

1. Verbindungen der Formel I
R¹-(CH₂)ₙ-(Y)_{q}-(Z)ᵣ-CO-NH-R² I
worin
R¹ unsubstituiertes oder ein- oder zweifach durch A, AO, OH, Hal, CN, NO₂, NH₂, NHA, NA₂, COA, CONH₂, CONHA, CONA₂, CH₂OH, CH₂OA, CH₂NH₂, CH₂NHA, CH₂NA₂, COOH und/oder COOA substituiertes 3-Indolyl,
R² 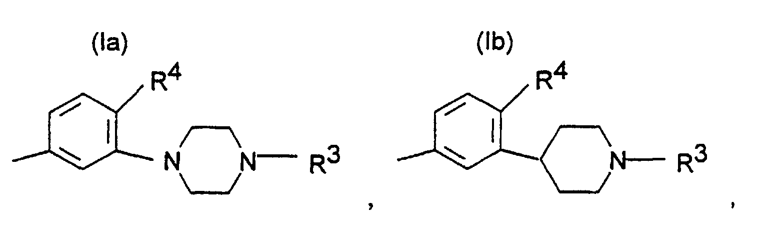 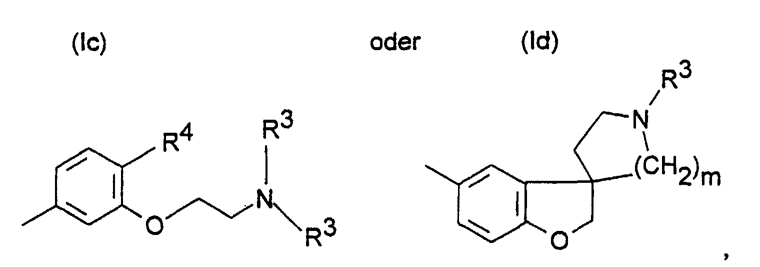
m 1 oder 2,
n 0, 1, 2, 3 oder 4,
Y einen 1,4-Cyclohexylen-, 1,3-Pyrrolidinylen-, 1,4-Piperazinylen- oder 1,4-Piperidinylenring, welcher auch teilweise dehydriert vorliegen kann,
Z (CH₂)ₙ oder (CH₂)ₙNH-,
q 0 oder 1,
r 0 oder 1,
R³ A,
R⁴ AO,
Hal F, Cl, Br oder I,
A geradkettiges oder verzweigtes Alkyl mit 1-6 C-Atomen,
bedeuten, mit der Maßgabe, dass q und r nicht gleichzeitig 0 sind, sowie deren physiologisch unbedenklichen Salze.

2. Verfahren zur Herstellung von Verbindungen der Formel I gemäß Anspruch 1, **dadurch gekennzeichnet, dass** man
a) eine Verbindung der Formel II
II H₂N-R²
worin R² die in Anspruch 1 angegebene Bedeutung hat,
mit einer Verbindung der Formel III
III R¹-(CH₂)ₙ-(Y)_{q}-(Z)ᵣ-CO-L
worin L Cl, Br, I, OH oder eine andere reaktionsfähig funktionell abgewandelte OH-Gruppe oder eine leicht nucleophil substituierbare Abgangsgruppe bedeutet und
R¹, n, Y, q, Z und r die in Anspruch 1 angegebenen Bedeutungen haben,
umsetzt,
oder
b) die Aminkomponente der Formel II
II H₂N-R²
mit der Komponente der Formel IV
IV R¹-(CH₂)ₙ-(Y)_{q}-(Z)ᵣ-H
worin R¹,R², n, Y, q, Z und r die angegebenen Bedeutungen haben, unter Zusatz von Kupplungsreagenzien wie N,N'-Carbonyldiimidazol, Diphosgen, Triphosgen oder auch Chlorameisensäureestem
umsetzt,
und/oder
c) dass man gegebenenfalls einen der Reste R¹, R³ und/oder R⁴ in einen anderen Rest R¹, R³ und/oder R⁴ umwandelt, indem man z.B. eine OA-Gruppe unter Bildung einer OH-Gruppe spaltet und/oder eine CN-, COOH- oder COOA-Gruppe derivatisiert und/oder dass man z.B. ein primäres oder sekundäres N-Atom alkyliert und/oder dass man eine erhaltene Base oder Säure der Formel I durch Behandeln mit einer Säure oder Base in eines ihrer Salze umwandelt.

3. Verfahren zur Herstellung pharmazeutischer Zubereitungen, **dadurch gekennzeichnet, dass** man eine Verbindung der Formel 1 und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoffen in eine geeignete Dosierungsform bringt.

4. Verbindungen der Formel I nach Anspruch 1 und/oder ihre physiologisch unbedenklichen Salze als 5-HT_{1B/D}-Antagonisten mit 5-HT-reuptake-hemmender Wirkung.

5. Verbindungen der Formel I nach Anspruch 1 und/oder ihre physiologisch unbedenklichen Salze als Antidepressiva und Anxiolytika.

6. Pharmazeutische Zubereitung, **gekennzeichnet durch** einen Gehalt an mindestens einer Verbindung der allgemeinen Formel I und/oder einem ihrer physiologisch unbedenklichen Salze.

7. Verwendung von Verbindungen der Formel I nach Patentanspruch 1 oder von deren physiologisch unbedenklichen Salzen zur Herstellung eines Arzneimittels.

## Claims

1. Compounds of the formula I
R¹-(CH₂)ₙ-(Y)_{q}-(Z)ᵣ-CO-NH-R² I
in which
R¹ is 3-indolyl which is unsubstituted or monosubstituted or disubstituted by A, AO, OH, Hal, CN, NO₂, NH₂, NHA, NA₂, COA, CONH₂, CONHA, CONA₂, CH₂OH, CH₂OA, CH₂NH₂, CH₂NHA, CH₂NA₂, COOH and/or COOA,
R² is 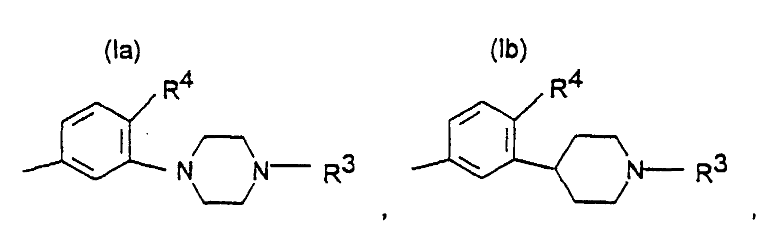 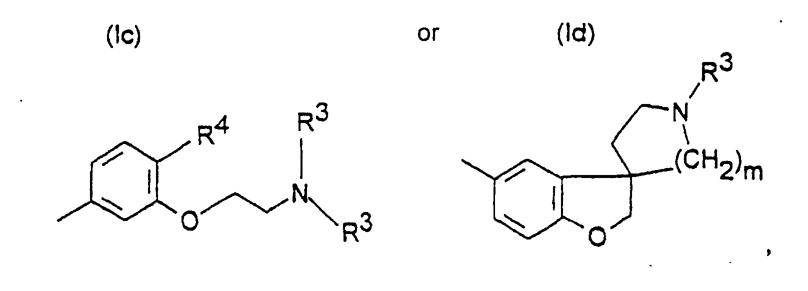
m is 1 or 2,
n is 0, 1, 2, 3 or 4,
Y is a 1,4-cyclohexylene, 1,3-pyrrolidinylene, 1,4-piperazinylene or 1,4-piperidinylene ring, which may also be in partially dehydrogenated form,
Z is (CH₂)ₙ or (CH₂)ₙNH-,
q is 0 or 1,
r is 0 or 1,
R³ is A,
R⁴ is AO,
Hal is F, Cl, Br or I,
A is straight-chain or branched alkyl having 1-6 carbon atoms,
with the proviso that q and r are not simultaneously 0, and their physiologically acceptable salts.

2. Process for the preparation of compounds of the formula I according to Claim 1, **characterized in that**
a) a compound of the formula II
II H₂N-R²
in which R² is as defined in Claim 1,
is reacted with a compound of the formula III
III R¹-(CH₂)ₙ-(Y)_{q}-(Z)ᵣ-CO-L
in which L is Cl, Br, I, OH or another reactively functionally modified OH group or a readily nucleophilically substitutable leaving group, and
R¹, n, Y, q, Z and r are as defined in Claim 1,
or
b) the amine component of the formula II
II H₂N-R²
is reacted with the component of the formula IV
IV R¹-(CH₂)ₙ-(Y)_{q}-(Z)ᵣ-H
in which R¹, R², n, Y, q, Z and r are as defined above, with addition of coupling reagents, such as N,N'-carbonyldiimidazole, diphosgene, triphosgene or also chloroformic acid esters,
and/or
c) if desired, one of the radicals R¹, R³ and/or R⁴ is converted into another radical R¹, R³ and/or R⁴ by, for example, cleaving an OA group to form an OH group and/or derivatizing a CN, COOH or COOA group, and/or, for example, a primary or secondary N atom is alkylated and/or a resultant base or acid of the formula I is converted into one of its salts by treatment with an acid or base.

3. Process for the preparation of pharmaceutical preparations, **characterized in that** a compound of the formula I and/or one of its physiologically acceptable salts is converted into a suitable dosage form together with at least one solid, liquid or semi-liquid excipient or adjuvant and, if desired, in combination with one or more further active ingredients.

4. Compounds of the formula I according to Claim 1 and/or their physiologically acceptable salts as 5-HT_{1B/D} antagonists having a 5-HT reuptake-inhibiting action.

5. Compounds of the formula I according to Claim 1 and/or their physiologically acceptable salts as antidepressants and anxiolytics.

6. Pharmaceutical preparation, **characterized by** a content of at least one compound of the general formula I and/or one of its physiologically acceptable salts.

7. Use of compounds of the formula I according to Patent Claim 1 or of their physiologically acceptable salts for the preparation of a medicament.

## Revendications

1. Composés de formule I
R¹-(CH₂)ₙ-(Y)_{q}-(Z)ᵣ-CO-NH-R² I
dans laquelle
R¹ représente 3-indolyle, qui est non substitué ou monosubstitué ou disubstitué par A, AO, OH, Hal, CN, NO₂, NH₂, NHA, NA₂, COA, CONH₂, CONHA, CONA₂, CH₂OH, CH₂OA, CH₂NH₂, CH₂NHA, CH₂NA₂, COOH et/ou COOA,
R² représente 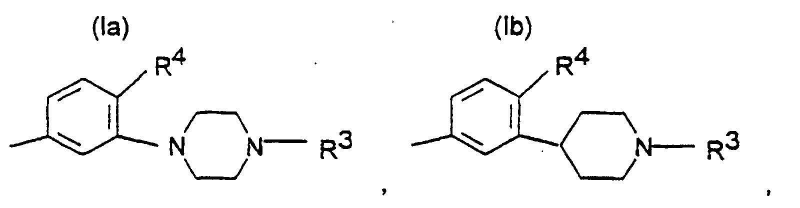 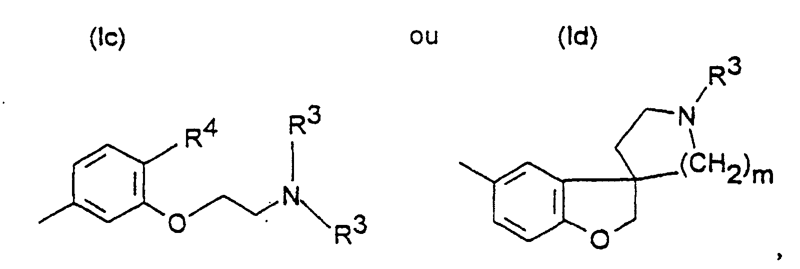
m vaut 1 ou 2,
n vaut 0, 1, 2, 3 ou 4,
Y représente un cycle 1,4-cyclohexylène, 1,3-pyrcolidinylène, 1,4-pipérazinylène ou 1,4-pipéridinylène, qui peut également être sous forme partiellement déshydrogénée,
Z représente (CH₂)ₙ ou (CH₂)ₙNH-,
q vaut 0 ou 1,
r vaut 0 ou 1,
R³ représente A,
R⁴ représente AO,
Hal représente F, Cl, Br ou I,
A représente un alkyle à chaîne linéaire ou ramifiée ayant 1-6 atomes de carbone,
avec la condition que q et r ne valent pas simultanément 0, et leurs sels physiologiquement acceptables.

2. Procédé de préparation de composés de formule I selon la revendication 1, **caractérisé en ce que**
a) un composé de formule II
II H₂N-R²
dans laquelle R² est tel que défini dans la revendication 1,
est réagi avec un composé de formule III
III R¹-(CH₂)ₙ-(Y)_{q}-(Z)ᵣ-CO-L
dans laquelle L représente Cl, Br, I, OH ou un autre groupement OH modifié fonctionnellement et réactivement ou un groupement partant pouvant être facilement substitué de manière nucléophile, et R¹, n, Y, q, Z et r sont tels que définis à la revendication 1,
ou
b) le composant amine de formule II
II H₂N-R²
est réagi avec le composant de formule IV
IV R¹-(CH₂)ₙ-(Y)_{q}-(Z)ᵣ-H
dans laquelle R¹, R², n, Y, q, Z et r sont tels que définis ci-dessus, avec l'addition d'agents de couplage, tels que le N,N'-carbonyldiimidazole, le diphosgène, le triphosgène ou encore des esters de l'acide chloroformique,
et/ou
c) si on le désire, l'un des radicaux R¹, R³ et/ou R⁴ est converti en un autre radical R¹, R³ et/ou R⁴, par exemple, par le clivage d'un groupement OA pour former un groupement OH et/ou la dérivatisation d'un groupement CN, COOH ou COOA, et/ou, par exemple, un atome de N primaire ou secondaire est alkylé et/ou une base ou un acide résultant(e) de formule I est converti(e) en l'un de ses sels par un traitement par un acide ou une base.

3. Procédé de préparation de préparations pharmaceutiques, **caractérisé en ce qu'**un composé de formule I et/ou l'un de ses sels physiologiquement acceptables est converti en une forme de dosage convenable avec au moins un excipient ou adjuvant solide, liquide ou semi-liquide et, si on le désire, en association avec un ou plusieurs ingrédients actifs supplémentaires.

4. Composés de formule I selon la revendication 1 et/ou leurs sels physiologiquement acceptables comme antagonistes du 5-HT_{1B/D} ayant une action d'inhibition du recaptage du 5-HT.

5. Composés de formule I selon la revendication 1 et/ou leurs sels physiologiquement acceptables, comme antidépresseurs et anxiolytiques.

6. Préparation pharmaceutique, **caractérisée par** une teneur en au moins un composé de formule générale I et/ou l'un de ses sels physiologiquement acceptables.

7. Utilisation de composés de formule I selon la revendication 1 ou de leurs sels physiologiquement acceptables, pour la préparation d'un médicament.
